# NEW EUROPEAN PATENT SPECIFICATION

(11) **EP 0 594 669 B2**
(45) Date of publication and mention of the opposition decision: **19.12.2007**
(45) Mention of the grant of the patent: 14.05.2003
(21) Application number: 92914286.7
(22) Date of filing: 07.07.1992
(51) Int. Cl.: C12N 5/00, C12N 5/06, A61K 35/30

(54) **Growth factor-responsive neural progenitor cells which can be proliferated in vitro.**
Auf Wachstumsfaktoren reagierende neurale Vorläuferzellen, die in vitro vermehrt werden können.
Cellules précurseurs neurales réagissant au facteur de croissance et capables de proliférer in vitro.

(30) Priority: 08.07.1991 US 726812
(43) Date of publication of application: 04.05.1994
(60) Divisional application: 03007791.1
(73) Proprietor: NeuroSpheres Holdings Ltd., Calgary, Alberta T2L 2K7 (CA)
(72) Inventor: WEISS, Samuel, Calgary, T2L 1A6, AL (CA); REYNOLDS, Brent, A., Calgary, T3B 2V6, AL (CA)
(74) Representative: Crump, Julian Richard John
(86) International application number: PCT/CA1992/000283
(87) International publication number: WO 1993/001275

(56) References cited:
- WO-A-91/09936
- SCIENCE, vol. 255, 27 March 1992, Lancaster, PA (US); B.A. REYNOLDS 'GENERATION OF NEURONS AND ASTROCYTES FROM ISOLATED CELLS OF THE ADULT MAMMALIAN CENTRAL NERVOUS SYSTEM'
- SCIENCE vol. 238, 2 October 1987, LANCASTER, PA US pages 72 - 75 RICHARD S. MORRISON ET AL. 'TROPHIC STIMULATION OF CULTURED NEURONS FROM NEONATAL RAT BRAIN BY EPIDERMAL GROWTH FACTOR'
- BIOLOGICAL ABSTRACTS vol. 92 , 1991, Philadelphia, PA, US; abstract no. 31828, MASTERS , BRIAN A. ET AL. 'INSULIN-LIKE GROWTH FACTOR I (IGF-I) RECEPTORS AND IGF-I ACTION IN OLIGODENDROCYTES FROM RAT BRAINS' & REGUL. PEPT. vol. 33, no. 2, 1991, pages 117-132
- BIOLOGICAL ABSTRACTS vol. 90 , 1990, Philadelphia, PA, US; abstract no. 78581, HUNTER, SAMUEL F. ET AL. 'GROWTH FACTOR RESPONSES OF ENRICHED BIPOTENTIAL GLIAL PROGENITORS' & DEV. BRAIN RES. vol. 54, no. 2, 1990, pages 235-248
- MEDLINE ABSTRACT 89111082 ABSTRACT & J NEUROSCI RES vol. 21, no. 2-4, October 1988, pages 210-219 SANETO R P ET AL. 'INSULIN/INSULIN-LIKE GROWTH FACTOR I AND OTHER EPIGENETIC MODULATORS OF MYELIN BASIC PROTEIN EXPRESSION IN ISOLATED OLIGODENDROCYTE PROGENITOR CELLS'
- Reynolds et al., Society of Neuroscience Abstracts, Vol. 15, Oct/Nov. 1990, abstract no. 474.2

## Description

This invention relates to a method for the in vitro culture of neural progenitor cells, and to the use of these cells as tissue grafts. In one aspect, this invention relates to a method for the isolation and in vitro perpetuation of large numbers of non-tumorigenic neural progenitor cells which can be induced to differentiate and which can be used for neurotransplantation into an animal to alleviate the symptoms of neurologic disease, neurodegeneration and central nervous system (CNS) trauma. In another aspect, this invention relates to a method of generating nerve cells for the purposes of drug screening of putative therapeutic agents targeted at the nervous system. In another aspect, this invention also relates to a method of generating cells for autologous transplantation. In another aspect, the invention relates to a method for the in situ proliferation and differentiation of the progenitor cells in the host.

The development of the nervous system begins at an early stage of fetal development. Neurogenesis, the generation of new neurons, is complete early in the postnatal period. However, the synaptic connections involved in neural circuits are continuously altered throughout the life of the individual, due to synaptic plasticity and cell death.

The first step in neural development is cell birth, which is the precise temporal and spatial sequence in which precursor or progenitor cells proliferate and differentiate. Proliferating cells will give rise to neuroblasts, glioblasts and stem cells.

The second step is a period of cell type differentiation and migration when progenitor cells become neurons and glial cells and migrate to their final positions. Cells which are derived from the neural tube give rise to neurons and glia of the central nervous system (CNS), while cells derived from the neural crest give rise to the cells of the peripheral nervous system (PNS). Certain factors present during development, such as nerve growth factor (NGF), promote the growth of neural cells. NGF is secreted by cells of the neural crest and stimulates the sprouting and growth of the neuronal axons.

The third step in development occurs when cells acquire specific phenotypic qualities, such as the expression of particular neurotransmitters. At this time, neurons also extend processes which synapse on their targets. Neurons do not divide subsequent to differentiation.

Finally, selective cell death occurs, wherein the degeneration and death of specific cells, fibers and synaptic connections "fine-tune" the complex circuitry of the nervous system. This "fine-tuning" continues throughout the life of the host. Later in life, selective degeneration due to aging, infection and other unknown etiologies can lead to neurodegenerative diseases.

The treatment of neurodegenerative diseases has become a major concern in recent years, due to the expanding elderly population which is at greatest risk for these diseases. These diseases, which include Alzheimer's Disease, Huntington's Chorea and Parkinson's Disease, have been linked to the degeneration of neurons in specific locations in the brain, which leads to the inability of the brain region to synthesize and release neurotransmitters which are vital to neuronal signalling. Another disease which is thought to arise from the lack of neurotransmitters is schizophrenia.

Neurodegeneration also encompasses those conditions and diseases which result in neuronal loss. These conditions can include those which arise as a result of CNS trauma, including stroke and epilepsy. Other diseases such as amyotrophic lateral sclerosis and cerebral palsy also result in neuronal loss. Because of a lack of understanding of the interactions among specific areas of the brain, and of how these interactions generate the outward manifestations of their functions, treatment of CNS degeneration is hampered. Studies of the intricate cellular interactions and their effect on behavior and cognitive function are made more difficult due to the large number of cells and the complex neural circuits which they form.

The objective of most CNS therapies is to regain the particular chemical function or enzymatic activity which is lost due to cellular degeneration.

Degeneration in a brain region known as the basal ganglia can lead to diseases with various cognitive and motor symptoms, depending on the exact location. The basal ganglia consists of many separate regions, including the striatum (which consists of the caudate and putamen), the globus pallidus, the substantia nigra, substantia innominata, ventral pallidum, nucleus basalis of Meynert, ventral tegmental area and the subthalamic nucleus.

In the case of Alzheimer's Disease, there is a profound cellular degeneration of the forebrain and cerebral cortex. In addition, upon closer inspection, a localized degeneration in an area of the basal ganglia, the nucleus basalis of Meynert, appears to be selectively degenerated. This nucleus normally sends cholinergic projections to the cerebral cortex which are thought to participate in cognitive functions including memory.

Many motor deficits are a result of degeneration in the basal ganglia. Huntington's Chorea is associated with the degeneration of neurons in the striatum, which leads to involuntary jerking movements in the host. Degeneration of a small region called the subthalamic nucleus is associated with violent flinging movements of the extremities in a condition called ballismus, while degeneration in the putamen and globus pallidus are associated with a condition of slow writhing movements or athetosis. In the case of Parkinson's Disease, degeneration is seen in another area of the basal ganglia, the substantia nigra par compacta. This area normally sends dopaminergic connections to the dorsal striatum which are important in regulating movement. Therapy for Parkinson's Disease has centered upon restoring dopaminergic activity to this circuit.

Other forms of neurological impairment can occur as a result of neural degeneration, such as amyotrophic lateral sclerosis and cerebral palsy, or as a result of CNS trauma, such as stroke and epilepsy.

To date, treatment for CNS dysfunction has been mainly via the administration of pharmaceutical compositions. Unfortunately, this type of treatment has been fraught with many complications including the limited ability to transport drugs across the blood-brain barrier, and the drug-tolerance which is acquired by patients to whom these drugs are administered long-term. For instance, partial restoration of dopaminergic activity in Parkinson's patients has been achieved with levodopa, which is a dopamine precursor able to cross the blood-brain barrier. However, patients become tolerant to the effects of levodopa, and therefore, steadily increasing dosages are needed to maintain its effects. There are presently no effective pharmaceutical treatments for Alzheimer's Disease.

Recently, the concept of neurological tissue grafting has been applied to the treatment of neurological diseases such as Parkinson's Disease. Neural grafts may avert the need not only for constant drug administration, but also for complicated drug delivery systems which arise due to the blood-brain barrier. However, there are limitations to this technique as well. First, cells used for transplantation which carry cell surface molecules of a differentiated cell from another host can induce an immune reaction in the host. In addition, the cells must be at a stage of development where they are able to form normal neural connections with neighboring cells. For these reasons, initial studies on neurotransplantation centered on the use of fetal cells. Perlow, et al. describe the transplantation of fetal dopaminergic neurons into adult rats with chemically induced nigrostriatal lesions in "Brain grafts reduce motor abnormalities produced by destruction of nigrostriatal dopamine system," Science 204: 643-647 (1979). These grafts showed good survival, axonal outgrowth and significantly reduced the motor abnormalities in the host animals. Lindvall, et al., in "Grafts of fetal dopamine neurons survive and improve motor function in Parkinson's Disease," Science 247: 574-577 (1990), showed that neural transplantation of human fetal mesencephalic dopamine neurons can restore dopamine synthesis and storage, and reduce rigidity and bradykinesia in patients suffering from Parkinson's disease. Freed, et al. in "Transplantation of human fetal dopamine cells for Parkinson's Disease," Arch. Neurol. 47: 505-512 (1990) also show improvement in a patient who received a fetal transplant.

The above references disclose that mammalian fetal brain tissue has good survival characteristics upon immediate transplantation. The increased survival capability of fetal neurons is thought to be due to the reduced susceptibility of fetal neurons to anoxia than adult neurons, and also to the lack of cell surface markers on fetal cells whose presence may lead to the rejection of grafted tissue from adults. However, although the brain is considered an immunologically privileged site, some rejection of fetal tissue can occur. Therefore, the ability to use fetal tissue is limited, not only due to tissue rejection of the fetal tissue isolated from another host, and because of the resultant need for immunosuppressant drugs, but also due to ethical problems in obtaining fetal tissue. However, neonatal brain tissue possesses limited capacity for survival and adult mammalian CNS neurons generally do not survive transplantation into the brain.

Although adult CNS neurons are not good candidates for neurotransplantation, neurons from the adult peripheral nervous system (PNS) have been shown to survive transplantation, and to exert neurotrophic and gliotrophic effects on developing host neural tissue. One source of non-CNS neural tissue for transplantation is the adrenal medulla. Adrenal chromaffin cells originate from the neural crest like PNS neurons, and receive synapses and produce carrier and enzyme proteins similar to PNS neurons. Although these cells function in an endocrine manner in the intact adrenal medulla, in culture these cells lose their glandular phenotype and develop neural features in culture in the presence of certain growth factors and hormones (Notter, et al., "Neuronal properties of monkey adrenal medulla in vitro, Cell Tissue Research 244: 69-76 [1986]). When grafted into mammalian CNS, these cells survive and synthesize significant quantities of dopamine which can interact with dopamine receptors in neighboring areas of the CNS.

In U.S. Patent No. 4,980,174, transplantation of monoamine-containing cells isolated from adult rat pineal gland and adrenal medulla into rat frontal cortex led to the alleviation of learned helplessness, a form of depression in the host. In U.S. Patent No. 4,753,635, chromaffin cells and adrenal medullary tissue derived from steers were implanted into the brain stem or spinal cord of rats and produced analgesia when the implanted tissue or cell was induced to release nociceptor interacting substances (i.e. catecholamines such as dopamine). Adrenal medullary cells have been autologously grafted into humans, and have survived, leading to mild to moderate improvement in symptoms (Watts, et al., "Adrenal-caudate transplantation in patients with Parkinson's Disease (PD):1-year follow-up," Neurology 39 Suppl 1: 127 [1989], Hurtig, et al., "Post-mortem analysis of adrenal-medulla-to-caudate autograft in a patient with Parkinson's Disease," Annals of Neurology 25: 607-614 [1989]). However, adrenal cells do not obtain a normal neural phenotype, and are therefore probably of limited use for transplants where synaptic connections must be formed.

Another source of tissue for neurotransplantation is from cell lines. Cell lines are immortalized cells which are derived either by transformation of normal cells with an oncogene (Cepko, "Immortalization of neural cells via retrovirus-mediated oncogene transduction," Ann. Rev. Neurosci. 12: 47-65 [1989]) or by the culturing of cells with altered growth characteristics in vitro (Ronnett, et al., "Human cortical neuronal cell line: Establishment from a patient with unilateral megalencephaly," Science 248: 603-605 [1990]). Such cells can be grown in culture in large quantities to be used for multiple transplantations. Some cell lines have been shown to differentiate upon chemical treatment to express a variety of neuronal properties such as neurite formation, excitable membranes and synthesis of neurotransmitters and their receptors. Furthermore, upon differentiation, these cells appear to be amitotic, and therefore non-cancerous. However, the potential for these cells to induce adverse immune responses, the use of retroviruses to immortalize cells, the potential for the reversion of these cells to an amitotic state, and the lack of response of these cells to normal growth-inhibiting signals make cell lines less than optimal for widespread use.

Reynolds et al., Soc. Neurosci. Abstracts, Vol. 15, Oct./Nov. 1990, Abstr. No. 474.2 disclose the effects of EGF or TGF-α in a primary culture of embryonic CNS progenitor cells.

Another approach to neurotransplantation involves the use of genetically engineered cell types or gene therapy. Using this method, a foreign gene or transgene can be introduced into a cell which is deficient in a particular enzymatic activity, thereby restoring activity. Cells which now contain the transferred gene can be transplanted to the site of neurodegeneration, and provide products such as neurotransmitters and growth factors (Rosenberg, et al., "Grafting genetically modified cells to the damaged brain: Restorative effects of NGF Expression," Science 242: 1575-1578, [1988]) which may function to alleviate some of the symptoms of degeneration. However, there still exists a risk of inducing an immune reaction with these cells and the retrovirus-mediated transfer may result in other cellular abnormalities. Also, cell lines produced by retrovirus-mediated gene transfer have been shown to gradually inactivate their transferred genes following transplantation (Palmer, et al., "Genetically modified skin fibroblasts persist long after transplantation but gradually inactivate introduced genes," Proc. Natl. Acad. Sci. 88: pp. 1330-1334 [1991]). In addition, these cells may also not achieve normal neuronal connections with the host tissue.

The inability in the prior art of the transplant to fully integrate into the host tissue, and the lack of availability of cells in unlimited amounts from a reliable source for grafting are, perhaps, the greatest limitations of neurotransplantation.

Therefore, in view of the aforementioned deficiencies attendant with prior art methods of neural^{•} cell culturing and transplantation, it should be apparent that there still exists a need in the art for a reliable source of unlimited numbers of cells for neurotransplantation, which are capable of differentiating into neurons. Furthermore, given that heterologous donor cells may be immunologically rejected, there exists a need for the isolation, perpetuation and transplantation of autologous progenitors from the juvenile or adult brain that are capable of differentiating into neurons. In addition, there exists a need for the repair of damaged neuronal tissue in a relatively non-invasive fashion, that is by inducing progenitor cells to proliferate and differentiate into neurons in situ.

Accordingly, a major object of the present invention is to provide a reliable source of an unlimited number of cells for neurotransplantation, which are capable of differentiating into neurons.

It is another object of the present invention to provide a method for the in vitro proliferation of neural progenitor cells from, juvenile and adult brain, for differentiation of these progenitors into neurons, and for neurotransplantation of the progenitor cells into a heterologous or autologous host. Progenitor cells may also be induced to proliferate and differentiate in situ thereby averting the need for transplantation.

A still further object of the invention is to provide a method of generating nerve cells for the purpose of screening putative therapeutic agents targeted at the nervous system.

As disclosed herein, the present invention relates to a method for the production *in vitro* of a cell culture of multipotent neural stem cells, said method comprising the steps of: (a) preparing or providing an initial culture medium comprising one or more growth factors capable of inducing multipotent neural stem cell proliferation said one or more growth factors being selected from the group consisting of acidic fibroblast growth factor, basic fibroblast growth factor, epidermal growth factor, an EGF- like ligand, amphiregulin and transforming growth factor alpha; (b) preparing a cell culture by adding to the culture medium of step (a) cells derived from mammalian neural tissue said mammalian tissue not being obtained from an human embryo, and containing at least one multipotent neural stem cell capable of dividing without limit and producing progeny that are capable of differentiating into neurons and glia; (c) proliferating said multipotent neural stem cell in said cell culture medium to produce neural stem cell progeny which includes daughter multipotent neural stem cells and daughter cells which are capable of differentiating into neurons and glia; and (d) transferring said neural stem cell progeny to fresh culture medium containing one or more growth factors capable of inducing multipotent neural stem cell proliferation said one or more growth factors being selected from the group consisting of acidic fibroblast growth factor, basic fibroblast growth factor, epidermal growth factor, an EGF- like ligand, amphiregulin and transforming growth factor alpha to proliferate said daughter multipotent neural stem cells and to produce more neural stem cell progeny.

In a preferred embodiment, the invention provides for a method for the production *in vitro* of a cell culture of multipotent neural stem cells as described herein above, wherein the proliferation of said multipotent neural stem cell in step (c) or said daughter multipotent neural stem cells in step (d) occurs in a suspension culture or in a substrate-attached culture.
Furthermore, the invention provides for a method as described herein above wherein said method comprises perpetuation of multipotent neural stem cells by reinitiating proliferation of the daughter multipotent neural stem cells in step (d) by repeating step (d).

In a preferred embodiment of the method of the invention said mammalian neural tissue comes from a human but is not obtained from an human embryo. Most preferably, said mammalian neural tissue comes from a fetus, neonate, juvenile or adult.

In another embodiment, the present invention provides for a method for producing *in vitro* differentiated neural cells derived from the neural stem cell progeny produced by the inventive method as described herein above comprising inducing said daughter cells which are capable of differentiating to differentiate by plating said daughter cells on a fixed substrate or by allowing said daughter cells which are capable of differentiating to differentiate in suspension without reinitiating proliferation.
The invention also provides for a method for drug screening of a putative therapeutic agent comprising applying said agent to the cell culture produced by the inventive method described herein above and monitoring the response of said cell culture.

Furthermore, the invention relates to a cell culture produced by the method as disclosed herein for use in treating neurological or neurodegenerative disease or CNS trauma, wherein said cell culture is derived from a source autologous to the intended recipient.
The invention also provides for the use of a cell culture produced by the inventive method described herein in the manufacture of a medicament for treating neurological or neurodegenerative disease or CNS trauma.
Preferably, said cell culture is derived from a source autologous to the intended recipient.
In another embodiment, the present invention relates to the use of a cell culture of the invention and described herein above wherein said neurological or neurodegenerative disease is Parkinson's Disease, Alzheimer's Disease, Huntington's Chorea, schizophrenia, amyotrophic lateral sclerosis, epilepsy, cerebral palsy or stroke or the neurodegenerative disease that causes ballismus or athetosis.

The invention discloses a method for the in vitro proliferation and differentiation of progenitor cells, comprising the steps of (a) isolating the cell from a mammal, (b) exposing the cell to a culture medium containing a growth factor, (c) inducing the cell to proliferate, and (d) inducing the cell to differentiate. Proliferation and perpetuation of the progenitor cell can be carried out either in suspension cultures, or by allowing cells to adhere to a fixed substrate. Proliferation and differentiation can be done before or after transplantation, and in various combinations of in vitro or in vivo conditions, including (1) proliferation and differentiation in vitro, then transplantation, (2) proliferation in vitro, transplantation, then further proliferation and differentiation in vivo, and (3) proliferation in vitro, transplantation and differentiation in vivo. The invention also discloses the proliferation and differentiation of the progenitor cells in situ, which can be done directly in the host without the need for transplantation.

The invention further discloses preservation of progenitor cells by cryogenesis.

The invention also discloses a method for the in vivo transplantation of a progenitor cell, treated as in any of (1) through (3) above, which comprises implanting, into a mammal, these cells which have been treated with at least one growth factor.

Furthermore, the invention discloses a method for treating neurodegenerative diseases comprising administering to a mammal progenitor cells which have been treated as in any of (1) through (3), have been treated with a growth factor and induced to differentiate into neurons. The invention also discloses a method for treating neurodegenerative disease comprising stimulating in situ mammalian CNS progenitor cells to proliferate and differentiate into neurons.

The invention also discloses a method for the transfection of these progenitor cells with vectors which can express the gene products for growth factors, growth factor receptors, and peptide neurotransmitters, or express enzymes which are involved in the synthesis of neurotransmitters, including those for amino acids, biogenic amines and neuropeptides, and for the transplantation of these transfected cells into regions of neurodegeneration.

The invention further discloses a method for the screening of potential neurologically therapeutic pharmaceuticals.

With the foregoing and other objects, advantages and features of the invention that will become hereinafter apparent, the nature of the invention may be more clearly understood by reference to the following detailed description of the invention and to the appended claims.

### BRIEF DESCRIPTION OF THE DRAWINGS

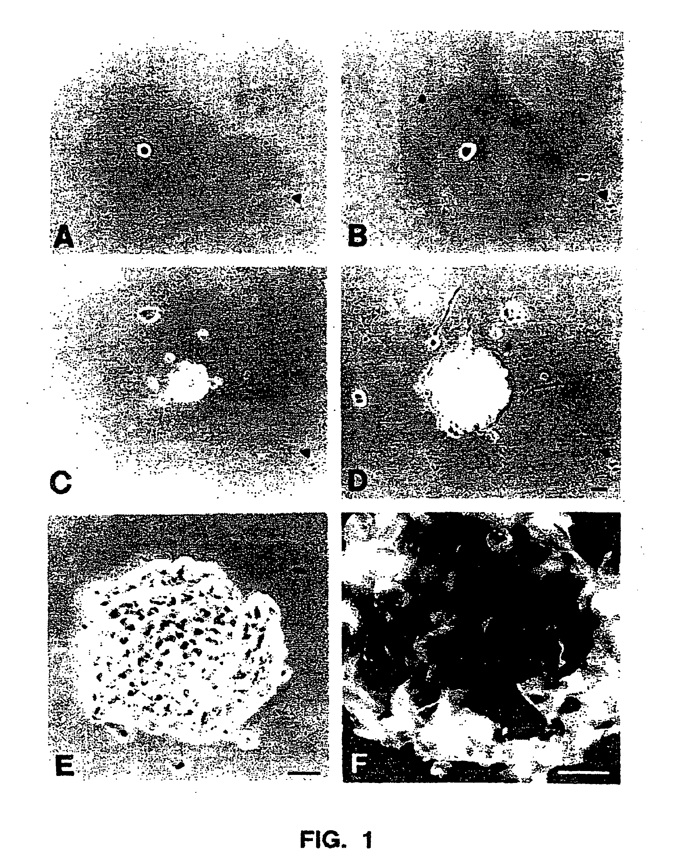

Figure 1. EGF-induced development of a cluster of undifferentiated cells from a single cell. With limiting dilution of the cell suspension, single cells were plated and identified at the base of 96-well plates. (A-D). Phase contrast micrographs of a single cell (A) after 3 days in vitro (DIV). The same cell began to divide after 5 DIV (B) and generated a cluster of cells observed after 7 (C) and 10 (D) DIV. Scratches in the plastic (arrowhead) serve to identify the field. Bar, 10µm. (E-F). Phase contrast micrograph (E) of 10 DIV cluster of cells, generated in response to EGF. The majority of cells in this cluster contained nestin-IR (F). Bar, 10 µm.

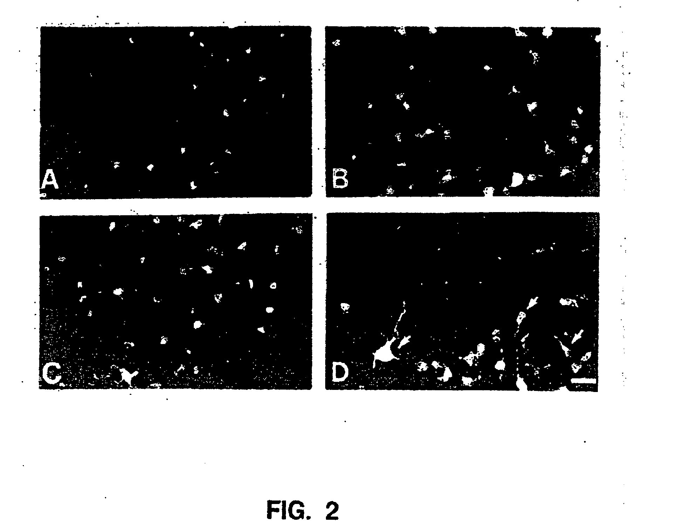

Figure 2. NSE-IR cells in EGF- and TGFα-induced proliferating cell clusters. The presence of NSE-IR cells in EGF and TGFα-induced proliferating clusters was examined at 20 and 25 DIV. Prior to 15 DIV no NSE-IR cells were detected. NSE-IR cells appeared after 16-18 DIV and were seen initially in the proliferating core. (A,B). After 20 DIV, numerous NSE-IR cells were identified in both EGF(A)-and TGFα(B)-treated cells. Few NSE-IR cells were found outside the proliferating core and no differences were apparent between the NSE-IR cells in the EGF- and TGFα-treated cultures. (C,D). After 25 DIV hundreds of NSE-IR cells were observed. In addition to large numbers of NSE-IR cells in the proliferating core, many IR cells had migrated away. At this stage, some differences in neuronal morphology between (C) EGF- and (D) TGFα-treated cells became apparent. While EGF-treated cells had short neurites, some of the NSE-IR cells in the TGFα-treated cultures had developed fairly long processes (arrows). Bar, 20µm.

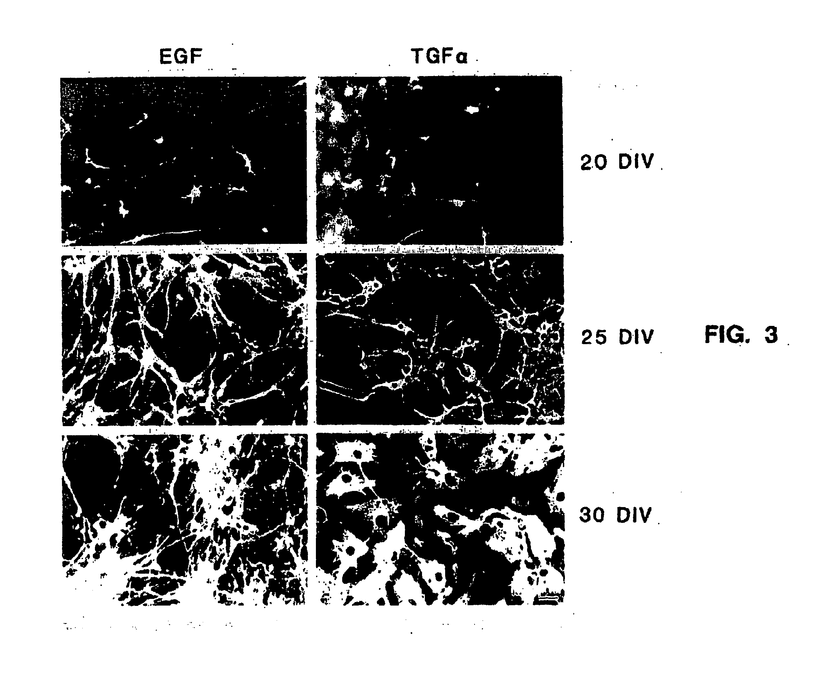

Figure 3. Appearance of GFAP-IR cells in EGF- and TGFα-treated cultures. GFAP-IR first appeared at 16-18 DIV. By 20 DIV a few GFAP-IR cells were observed migrating away from the proliferating core. There was no apparent difference between EGF- and TGFα-treated cultures. At 25 DIV, a large majority of the cells which had migrated away from the central core were GFAP-IR. At this stage all of the GFAP-IR cells in EGF-treated cultures had a stellate morphology, while many of the GFAP-IR in the TGFα-treated cultures assumed the morphology of immature protoplasmic astrocytes (some stellate cells were still apparent). By 30 DIV, all of the GFAP-IR cells in the EGF-treated cultures exhibited stellate morphology. In contrast, nearly all of the GFAP-IR cells in the TGFα-treated cultures were protoplasmic. Bar, 20µm.

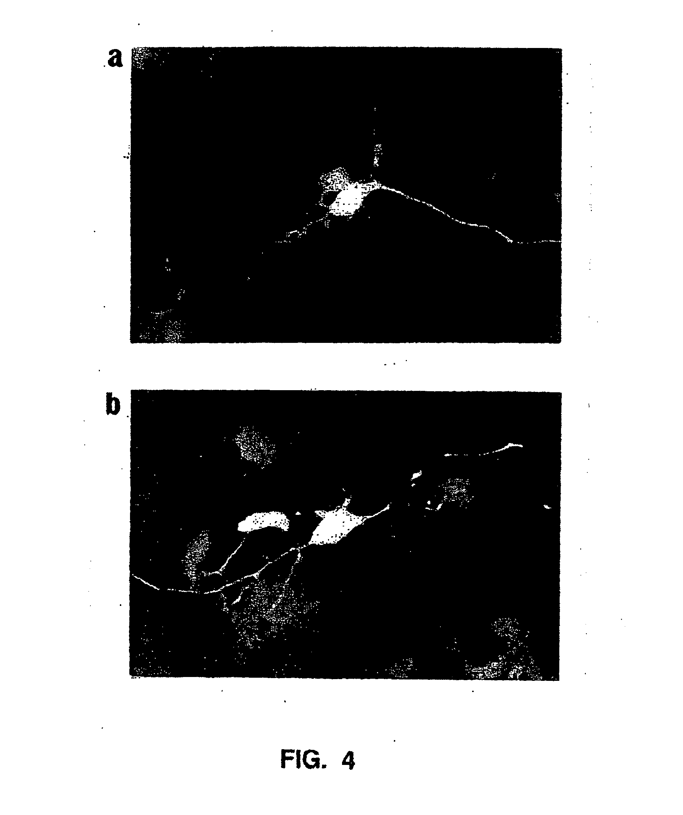

Figure 4. Phenotype of neurons generated by EGF-induced proliferation of adult striatal progenitor cells. Fluorescence micrographs of cells with neuronal morphology, in 21 DIV cultures of a single EGF-generated sphere plated on a fixed substrate, after staining for GABA (a) and substance P (b).

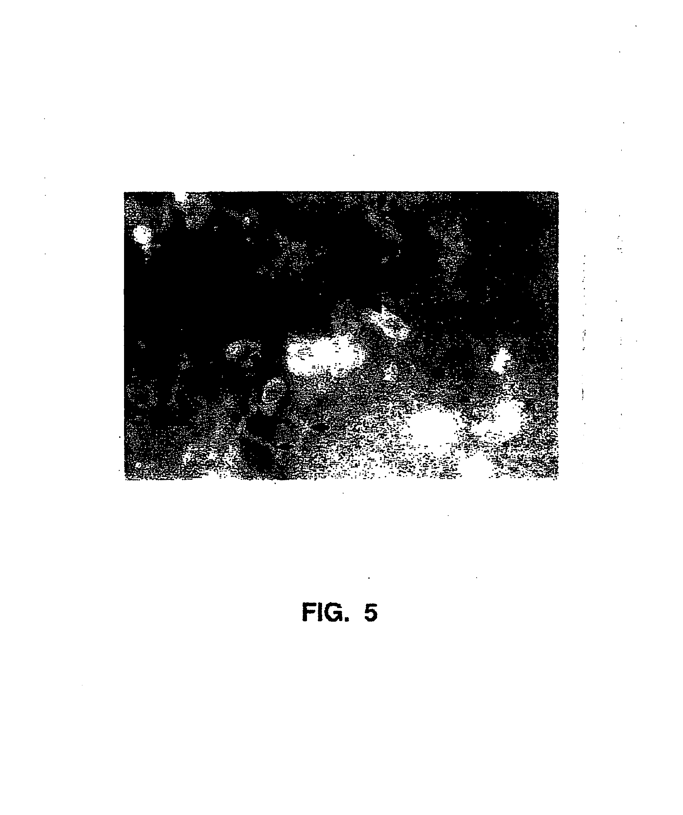

Figure 5. Transplanted mouse neurospheres differentiate into neurons in the rat CNS. A monoclonal antibody, M6, which recognizes a surface protein found only on mouse neurons was used to label transplanted mouse progenitors which had differentiated into neurons in the rat host. Positive labelled cell bodies (large arrows) and neurites (small arrows) are identified.

The present invention provides for the isolation of neural progenitor cells from an animal, for perpetuation of these cells in vitro or in vivo in the presence of growth factors, for the differentiation of these cells into neurons and glia, for the cryogenesis of progenitor cells, and for the use of these cells for neurotransplantation into a host in order to treat neurodegenerative disease and neurological trauma, and for drug-screening applications. The induction of proliferation and differentiation in these progenitor cells can be done either by growing adherent or suspension cells in tissue culture, or, under appropriate conditions, in the host in the following combinations: (1) proliferation and differentiation in vitro, then transplantation, (2) proliferation in vitro, transplantation, then further proliferation and differentiation in vivo, (3) proliferation in vitro, transplantation and differentiation in vivo, and (4) proliferation and differentiation in situ. Proliferation and differentiation in situ involves a non-surgical approach which coaxes progenitor cells to proliferate in situ with pharmaceutical manipulation.

Progenitor cells are thought to be under a tonic inhibitory influence which maintains the progenitors in a suppressed state until their differentiation is required. This tonic inhibitory influence may be a soluble factor, as fractions of neural lysates added back to progenitor cells can inhibit their proliferation in vitro. In culture, these progenitor cells may not be subject to this same inhibitory influence. These cells are now able to be proliferated, and unlike neurons which are terminally differentiated and therefore non-dividing, they can be produced in unlimited number and are therefore highly suitable for transplantation into heterologous and autologous hosts with neurodegenerative diseases.

By progenitor is meant an oligopotent or multipotent stem cell which is able to divide without limit and under specific conditions can produce daughter cells which terminally differentiate into neurons and glia. These cells can be used for transplantation into a heterologous or autologous host. By heterologous is meant a host other than the animal from which the progenitor cells were originally derived. By autologous is meant the identical host from which the cells were originally derived.

Cells can be obtained from post-natal, juvenile or adult neural tissue from any animal. By any animal is meant any multicellular animal which contains nervous tissue. More particularly, is meant any insect, fish, reptile, bird, amphibian or mammal and the like. The most preferable donors are mammals, especially mice and humans.

In the case of a heterologous donor animal, the animal may be euthanized, and the brain and specific area of interest removed using a sterile procedure. Brain areas of particular interest include any area from which progenitor cells can be obtained which will serve to restore function to a degenerated area of the host's brain. These regions include areas of the central nervous system (CNS) including the cerebral cortex, cerebellum, midbrain, brainstem, spinal cord and ventricular tissue, and areas of the peripheral nervous system (PNS) including the carotid body and the adrenal medulla. More particularly, these areas include regions in the basal ganglia, preferably the striatum which consists of the caudate and putamen, or various cell groups such as the globus pallidus, the subthalamic nucleus, the nucleus basalis which is found to be degenerated in Alzheimer's Disease patients, or the substantia nigra pars compacta which is found to be degenerated in Parkinson's Disease patients. This latter cell group is named for and identified by its dark color due to the presence of high levels of the pigment, melanin. Melanin is derived from tyrosine or dopa through the action of tyrosinase.

Human heterologous neural progenitor cells may be derived from fetal tissue obtained from elective abortion, or from a post-natal, juvenile or adult organ donor. Autologous neural tissue can be obtained by biopsy, or from patients undergoing neurosurgery in which neural tissue is removed, in particular during epilepsy surgery, and more particularly during temporal lobectomies and hippocampalectomies.

Cells can be obtained from donor tissue by dissociation of individual cells from the connecting extracellular matrix of the tissue. Dissociation can be obtained using any known procedure, including treatment with enzymes such as trypsin, collagenase and the like, or by using physical methods of dissociation such as with a blunt instrument. Dissociation of fetal cells can be carried out in tissue culture medium, while a preferable medium for dissociation of juvenile and adult cells is artificial cerebral spinal fluid (aCSF). Regular aCSF contains 124 mM NaCl, 5 mM KCl, 1.3 mM MgCl₂, 2 mM CaCl₂, 26 mM NaHCO₃, and 10 mM D-glucose. Low Ca⁺⁺ aCSF contains the same ingredients except for MgCl₂ at a concentration of 3.2 mM and CaCl₂ at a concentration of 0.1 mM.

Dissociated cells can be placed into any known culture medium capable of supporting cell growth, including MEM, DMEM, RPMI, F-12, and the like, containing supplements which are required for cellular metabolism such as glutamine and other amino acids, vitamins, minerals and useful proteins such as transferrin and the like. Medium may also contain antibiotics to prevent contamination with yeast, bacteria and fungi 'such as penicillin, streptomycin, gentamicin and the like. In some cases, the medium may contain serum derived from bovine, equine, chicken and the like. A particularly preferable medium for cells is a mixture of DMEM and F-12.

Conditions for culturing should be close to physiological conditions. The pH of the culture media should be close to physiological pH, preferably between pH 6-8, more preferably close to pH 7, even more particularly about pH 7.4. Cells should be cultured at a temperature close to physiological temperature, preferably between 30°C-40°C, more preferably between 32°C-38°C, and most preferably between 35°C-37°C.

Cells can be grown in suspension or on a fixed substrate, but proliferation of the progenitors is preferably done in suspension to generate large numbers of cells. In the case of suspension cells, flasks are shaken well and the neurospheres allowed to settle on the bottom corner of the flask. The spheres are then transferred to a 50 ml centrifuge tube and centrifuged at low speed. The medium is aspirated, the cells resuspended in a small amount of medium with growth factor, and the cells mechanically dissociated. The cells are then counted and replated.

Culturing of cells on a fixed substrate can lead to differentiation of the progenitor into a terminally differentiated cell which is no longer capable of dividing, but can be used for transplantation to form connections with other neural targets. Differentiation can also be induced in suspension if the cells are cultured for a period of time without dissociation and re-initiation of proliferation.

Growth factors which may be used for inducing proliferation include any trophic factor which allows progenitor cells to proliferate, including any molecule which binds to a receptor on the surface of the cell to exert a trophic, or growth-inducing effect on the cell. Such factors include acidic and basic fibroblast growth factor (aFGF, bFGF), epidermal growth factor (EGF), an EGF-like ligand, amphiregulin and transforming growth factor alpha (TGFα).

The most preferable factors are EGF and TGFα. These growth factors are single chain polypeptides of 53 and 50 amino acids, respectively, which compete for binding of the EGF receptor (EGFR). EGF and TGFα have been demonstrated to influence cellular differentiation. TGFα immunoreactivity (IR), EGF-IR and EGFR-IR, as well as TGFα and EGF mRNAs have been observed in the rodent and human CNS. The results of ligand binding studies have demonstrated that the number of binding sites for [¹²⁵I]EGF in the rodent brain increase during gestation and are higher in the first post-natal week than in the adult, consistent with a role for this factor in CNS development. EGF induces the proliferation of a multipotent progenitor cell from the striatum that gives rise to stellate astrocytes and neurons with short processes, and TGFα induces the proliferation of a multipotent progenitor cell that gives rise to protoplasmic astrocytes and neurons with long, well developed neurites. The concentration of growth factors used is 1 fg/ml - 1 mg/ml, preferably 20 ng/ml.

Neuronal and glial cells can be derived following the differentiation of the multipotent progenitor cells. Differentiation of the cells can be induced by any method known in the art which activates the cascade of biological events which lead to growth, which include the liberation of inositol triphosphate and intracellular Ca⁺⁺, liberation of diacyl glycerol and the activation of protein kinase c and other cellular kinases, and the like. Treatment with phorbol esters, growth factors and other chemical signals, as well as growth on a fixed substrate such as an ionically charged surface such as poly-L-lysine and poly-L-ornithine and the like can induce differentiation. Differentiation can also be induced by leaving the cells in suspension in the continued presence of growth factor, in the absence of reinitiation of proliferation.

Cells from the particular neural region are removed from the animal using a sterile procedure, and are mechanically dissociated using any method known in the art. Early post-natal brain tissue is dissociated directly into culture medium, while juvenile or adult brain tissue are dissociated into artificial cerebral spinal fluid (CSF). Dissociated cells are centrifuged at-low speed, between 200 and 2000 rpm, and more particularly between 400 and 800 rpm, and then resuspended in culture medium. Cells are resuspended at a approximately 5 x 10⁴ to 2 x 10⁵ cells/ml, preferably 1 x 10⁵ cells/ml. Cells plated on a fixed substrate are plated at approximately 2-3 x 10³ cells/cm², preferably 2.5 x 10³ cells/cm².

Cell suspensions in culture medium are supplemented with any growth factor which allows for the proliferation of progenitor cells and seeded in any receptacle capable of sustaining cells, particularly culture flasks, culture plates or roller bottles, and more particularly in small culture flasks such as 25 cm² culture flasks. Cells proliferate within 3-4 days in a 37°C incubator, and proliferation can be reinitiated at any time after that by dissociation of the cells and resuspension in fresh medium containing growth factors.

In the absence of substrate, cells lift off the floor of the flask and continue to proliferate in. suspension forming a hollow sphere of undifferentiated cells. After approximately 3-10 days in vitro, and more particularly approximately 6-7 days in vitro, the proliferating clusters (neurospheres) are fed every 2-7 days, and more particularly every 2-4 days by gentle centrifugation and resuspension in medium containing growth factor.

After 6-7 days in vitro, individual cells in the neurospheres can be separated by physical dissociation of the neurospheres with a blunt instrument, more particularly by triturating the neurospheres with a pipette, especially afire polished pasteur pipette. Single cells from the dissociated neurospheres are suspended in culture medium containing growth factor, and a percentage of these cells proliferate and form new neurospheres largely composed of undifferentiated cells. Differentiation of cells can be induced by plating the cells on a fixed substrate, including poly-L-lysine or poly-L-ornithine coated flasks, plates or coverslips in the continued presence of EGF, TGFα or any factor capable of sustaining differentiation such as basic fibroblast growth factor (bFGF) and the like. Differentiation can also be induced by leaving the cells in suspension in the presence of a growth factor, including EGF or TGFα, without reinitiation of proliferation.

In order to identify cellular molecules on the two types of cells which arise upon growth-factor-induced differentiation, namely neurons and glia, immunocytochemistry is performed using antibodies specific for known cellular markers. Antibodies specific for any neuronal or glial proteins can be used to distinguish the cellular characteristics or phenotypic properties of neurons from glia. In particular these cellular markers include, for neurons, neuron specific enolase (NSE) and neurofilament, and for glia, glial fibrillary acidic protein (GFAP), galactocerebroside and the like.

Also useful for identifying neuronal cells are antibodies against neurotransmitters. These include antibodies to acetylcholine (ACh), dopamine (DA), epinephrine (E), norepinephrine (NE), histamine (H), serotonin or 5-hydroxytryptamine (5-HT), neuropeptides such as substance P (SP), adrenocorticotrophic hormone (ACTH), vasopressin or anti-diuretic hormone (ADH), oxytocin, somatostatin, angiotensin II, neurotensin, and bombesin, hypothalamic releasing hormones such as thyrotropic-releasing hormone (TRH) and luteinizing hormone releasing hormone (LHRH), gastrointestinal peptides such as vasoactive intestinal peptide (VIP) and cholecystokinin (CCK) and CCK-like peptide, opioid peptides such as endorphins like β-endorphin and enkephalins such as met- and leu-enkephalin, prostaglandins, amino acids such as gamma-aminobutyric acid (GABA), glycine, glutamate, cysteine, taurine and aspartate and dipeptides such as carnosine.

Antibodies to neurotransmitter-synthesizing enzymes are also useful such as glutamic acid decarboxylase (GAD) which is involved in the synthesis of GABA, choline acetyltransferase (CAT) for ACh synthesis, dopa decarboxylase (DDC) for dopamine, dopamine-β-hydroxylase (DBH) for NE, and amino acid decarboxylase (AADC) for 5-HT.

Antibodies to enzymes which are involved in the deactivation of neurotransmitters may also be useful such as acetyl cholinesterase (AChE) which deactivates ACh. Antibodies to enzymes involved in the reuptake of neurotransmitters into neuronal terminals such as monoamine oxidase (MAO) and catechol-o-methyl transferase (COMT) for DA, MAO for 5-HT, and GABA transferase (GABA-T) for GABA may also identify neurons.

Other markers for neurons include antibodies to neurotransmitter receptors such as the AChE nicotinic and muscarinic receptors, adrenergic receptors α₁, α₂, β₁ and β₂, the dopamine receptor and the like.

Cells which contain a high level of melanin, such as those which would be found in the substantia nigra, could be identified using an antibody to melanin.

Progenitor cells can be identified with antibodies which specifically bind to proteins found only in progenitor cells, and not cells which have differentiated. In particular are antibodies to an intermediate filament protein, and more particularly an antibody called Rat401 which is directed to nestin, an intermediate filament protein found specifically in neuroepithelial stem cells.

Any secondary antibody or heterologous anti-immunoglobulin, can be used which binds to the primary antibody, and which allows for its detection by being conjugated to a particular enzyme, isotope, particle or dye. Such antibodies can be raised in any animal, such as goats, sheep, rabbits, swine, horses, mice, rats and the like, and be directed to a primary antibody raised in any heterologous animal of the same such varieties. Secondary antibodies can be conjugated to dyes such as fluorescein, rhodamine or other dye, can be radiolabelled with any isotope such as ¹²⁵I or conjugated to a radiolabelled protein such as digoxin, or can be conjugated to particles which can be seen in the microscope such as immuno-gold particles and the like. Secondary antibodies can also be conjugated to any enzymes which can react with a substrate to form a visible or detectable reaction product, such as horseradish-peroxidase and alkaline phosphatase. Particularly useful antibodies are secondary antibodies consisting of fluorescein conjugated affinipure goat anti-mouse IgG and rhodamine conjugated affinipure goat anti-rabbit IgG.

Cells are cultured on any substrate on which cells can grow and differentiate, and which can be analyzed using microscopy, such as culture plates, glass slides, cover slips and the like. Cells are cultured up to 6 weeks, and more preferably for 14-30 days in vitro, and can be fixed in any dehydrating solution such as an alcohol or acetone, or in a solution of cross-linking aldehydes. Cells are then stained using primary antibodies directed to any particular cellular feature which is to be identified, followed by reaction with secondary conjugated antibodies for detection.

Transplanted cells can be administered to any animal with abnormal neurological or neurodegenerative symptoms obtained in any manner, including those obtained as a result of chemical or electrolytic lesions, as a result of experimental aspiration of neural areas, or as a result of aging processes. Particularly preferable lesions in non-human animals are obtained with 6-hydroxy-dopamine (6-OHDA), 1-methyl-4-phenyl-1,2,3,6-tetrahydropyridine (MPTP), ibotenic acid and the like.

Because immunosuppressants carry some risk of morbidity and mortality as a result of infections which may occur, only autologous transplantation or heterologous transplantation using tissue from parents or siblings, can be done in the absence of these drugs. Transplantation can be done bilaterally, or, in the case of a patient suffering from Parkinson's Disease, contralateral to the most affected side. Surgery is performed in a manner in which particular brain regions may be located, such as in relation to skull sutures, particularly with a stereotaxic guide. Cells are delivered throughout any affected neural area, in particular to the basal ganglia, and preferably to the caudate and putamen, the nucleus basalis or the substantia nigra. Cells are administered to the particular region using any method which maintains the integrity of surrounding areas of the brain, preferably by injection cannula.

Cells administered to the particular neural region preferably form a neural graft, wherein the cells form normal neuronal or synaptic connections with neighboring neurons, and maintain contact with glial cells which may form myelin sheaths around the neuron's axon, and provide a trophic influence for the neurons. As these transplanted cells form connections, they reestablish the neuronal networks which have been damaged due to disease and aging.

Progenitor cells can be induced to proliferate and differentiate in situ by administering to the host, any growth factor or pharmaceutical composition which will induce proliferation and differentiation of the cells. These growth factors include any growth factor known in the art, including the growth factors described above for in vitro proliferation and differentiation, and in particular EGF and TGFα. Pharmaceutical compositions include any substance which blocks the inhibitory influence and/or stimulate the progenitor to proliferate and ultimately differentiate. Administration of growth hormones can be done by any method, including injection cannula, transfection of cells with growth-hormone-expressing vectors, injection, timed-release apparati which can administer substances at the desired site, and the like. Pharmaceutical compositions can be administered by any method, including injection cannula, injection, oral administration, timed-release apparati and the like.

Survival of the graft in the living host can be examined using various non-invasive scans such as computerized axial tomography (CAT or CT scan), nuclear magnetic resonance or magnetic resonance imaging (NMR or MRI) or more preferably positron emission tomography (PET) scans. Post-mortem examination of graft survival can be done by removing the neural tissue, and examining the affected region macroscopically, or more preferably using microscopy. Cells can be stained with any stains visible under light or electron microscopic conditions, more particularly with stains which are specific for neurons and glia. Particularly useful are monoclonal antibodies which identify neuronal cell surface markers such as the M6 antibody which identifies mouse neurons. Most preferable are antibodies which identify any neurotransmitters, particularly those directed to gamma amino butyric acid (GABA) and substance P, and to enzymes involved in the synthesis of neurotransmitters, in particular, glutamic acid decarboxylase (GAD). Transplanted cells can also be identified by prior incorporation of tracer dyes such as rhodamine- or fluorescein-labelled microspheres, fast blue, bis-benzamide or retrovirally introduced histochemical markers such as the lac Z gene which produces beta galactosidase.

Functional integration of the graft into the host's neural tissue can be assessed by examining the effectiveness of grafts on restoring various functions, including but not limited to tests for endocrine, motor, cognitive and sensory functions. Motor tests which can be used include those which guantitate rotational movement away from the degenerated side of the brain, and those which quantitate slowness of movement, balance, coordination, akinesia or lack of movement, rigidity and tremors. Cognitive tests include various tests of ability to perform everyday tasks, as well as various memory tests, including maze performance.

In another embodiment of the invention, progenitor cells are transplanted into a host, and induced to proliferate and/or differentiate in that host by either (1) proliferation and differentiation in vitro, then transplantation, (2) proliferation in vitro, transplantation, then further proliferation and differentiation in vivo, or (3) proliferation in vitro, transplantation and differentiation in vivo. Alternatively, the cells can be induced to proliferate and differentiate in situ by induction with particular growth factors or pharmaceutical compositions which will induce their proliferation and differentiation. Therefore, this latter method is non-surgical, and thus circumvents the problems associated with physical intervention and immune reactions to foreign cells. Any growth factor can be used, particularly EGF, TGFα, aFGF, and bFGF. These growth factors can be administered in any manner known in the art in which the factors may either pass through or by-pass the blood-brain barrier (BBB).

Methods for allowing factors to pass through the blood-brain barrier include minimizing the size of the factor, or providing hydrophobic factors which may pass through more easily.

Methods for by-passing the blood-brain barrier include transfection of the progenitor cells with expression vectors containing genes which code for growth factors, so that the cells themselves produce the factor. Cells can be transfected by any means known in the art, including CaPO₄ transfection, DEAE-dextran transfection, polybrene transfection, by protoplast fusion, electroporation, lipofection, and the like.

Any expression vector known in the art can be used to express the growth factor, as long as it has a promoter which is active in the cell, and appropriate termination and polyadenylation signals. These expression vectors include recombinant vaccinia virus vectors including pSC11, or vectors derived various viruses such as from Simian Virus 40 (SV40, i.e. pSV2-dhfr, pSV2neo, pko-neo, pSV2gpt, pSVT7 and pBABY), from Rous Sarcoma Virus (RSV, i.e. pRSVneo), from mouse mammary tumor virus (MMTV, i.e. pMSG), from adenovirus (pMT2), from herpes simplex virus (HSV, i.e. pTK2 and pHyg), from bovine papillomavirus (BPV, i.e. pdBPV and pBV-1MTHA), from Epstein-Barr Virus (EBV, i.e. p205 and pHEBo) or any other eukaryotic expression vector known in the art.

Any growth factor known to induce proliferation, growth or differentiation of the progenitor cells can be used for expression in the progenitor cells, including FGF, EGF and TGFα. Cells may also be transformed with receptors for any growth factor such as for bFGF, and EGF. In addition, cells may be transfected with any gene coding for a neurotransmitter or neurotransmitter-synthesizing enzyme which was listed above or any other for which expression in the host is desired.

Other methods for providing growth factors to the area of transplantation include the implantation into the brain in proximity to the graft of any device which can provide an infusion of the factor to the surrounding cells.

Progenitor cells can be cryopreserved by any method known in the art, by suspending the cells in an isotonic solution, preferably a cell culture medium, containing a particular cryopreservant. Such cryopreservants include dimethyl sulfoxide (DMSO), glycerol and the like. These cryopreservants are used at a concentration of 5-15%, preferably 8-10%. Cells are frozen gradually to a temperature of -10°C to -150°C, preferably -20°C to -100°C, and more preferably -70°C to -80°C.

Progenitor cells cultured in vitro can be used for the screening of potential neurologically therapeutic compositions. These compositions can be applied to cells in culture at varying dosages, and the response of the cells monitored for various time periods. Physical characteristics of the cells can be analyzed by observing cell and neurite growth with microscopy. The induction of expression of new or increased levels of proteins such as enzymes, receptors and other cell surface molecules, or of neurotransmitters, amino acids, neuropeptides and biogenic amines can be analyzed with any technique known in the art which can identify the alteration of the level of such molecules. These techniques include immunohistochemistry using antibodies against such molecules, or biochemical analysis. Such biochemical analysis includes protein assays, enzymatic assays, receptor binding assays, enzyme-linked immunosorbant assays (ELISA), electrophoretic analysis, analysis with high performance liquid chromatography (HPLC), Western blots, and radioimmune assays (RIA). Nucleic acid analysis such as Northern blots can be used to examine the levels of mRNA coding for these molecules, or for enzymes which synthesize these molecules. Alternatively, cells treated with these pharmaceutical compositions can be transplanted into an animal, and their survival, ability to form neuronal connections, and biochemical and immunological characteristics examined as described above.

The following examples are presented in order to more fully illustrate the preferred embodiments of the invention. They should in no way be construed, however, as limiting the scope of the invention, as defined by the appended claims.

### EXAMPLE 1

14-day-old CD₁ albino mouse embryos (Charles River) were decapitated and the brain and striata were removed using sterile procedure. Tissue was mechanically dissociated with a fire-polished Pasteur pipette into serum-free medium composed of a 1:1 mixture of Dulbecco's modified Eagle's medium (DMEM) and F-12 nutrient (Gibco). Dissociated cells were centrifuged at 800 r.p.m. for 5 minutes, the supernatant aspirated, and the cells resuspended in DMEM/F-12 medium for counting.

### EXAMPLE 2

Brain tissue from juvenile and adult mouse brain tissue was removed and dissected into 500 µm sections and immediately transferred into low calcium oxygenated artificial cerebrospinal fluid (low Ca⁺⁺ aCSF) containing 1.33 mg/ml trypsin, 0.67 mg/ml hyaluronidase, and 0.2 mg/ml kynurenic acid. Tissue was stirred in this solution for 90 minutes at 32°C-35°C. aCSF was poured off and replaced with fresh oxygenated aCSF for 5 minutes. Tissue was transferred to DMEM/F-12/10% hormone solution containing 0.7 mg/ml ovomucoid and triturated with a fire polished pasteur pipette. Cells were centrifuged at 400 r.p.m. for 5 minutes, the supernatant aspirated and the pelleted cells resuspended in DMEM/F-12/10% hormone mix.

### EXAMPLE 3

Dissociated cells prepared as in Examples 1 and 2 were diluted to approximately 1 cell/well in a 96 well (100 µl/well) tissue culture plate to examine the clonal nature of EGF-induced proliferation. The presence of a single cell in a well was confirmed with phase contrast microscopy.

### EXAMPLE 4

2500 cells/cm² prepared as in Example 1 were plated on poly-L-ornithine-coated (15 µg/ml;Sigma) glass coverslips in 24 well Nunclon (0.5 ml/well) culture dishes. The culture medium was a serum-free medium composed of DMEM/F-12 (1:1) including glucose (0.6%), glutamine (2 µM), sodium bicarbonate (3 mM), and HEPES (4-[2-hydroxyethyl]-1-piperazineethanesulfonic acid) buffer (5 mM) (all from Sigma except glutamine [Gibco]). A defined hormone mix and salt mixture (Sigma) that included insulin (25 µg/ml), transferrin (100 µg/ml), progesterone (20 nM), putrescine (60 µM), and selenium chloride (30 nM) was used in place of serum. Cultures contained the above medium together with 16-20 ng/ml EGF (purified from mouse submaxillary, Collaborative Research) or TGFα (human recombinant, Gibco). After 10-14 days in vitro, media (DMEM only plus hormone mixture) and growth factors were replaced. This medium change was repeated every two to four days. The number of surviving cells at 5. days in vitro was determined by incubating the coverslips in 0.4% trypan blue (Gibco) for two minutes, washing with phosphate buffered saline (PBS, pH 7.3) and counting the number of cells that excluded dye with a Nikon Diaphot inverted microscope.

### EXAMPLE 5

Dissociated mouse brain cells prepared as in Examples 1 and 2 (at 1 x 10⁵ cell/ml) were suspended in the culture medium described in Example 4 together with 20 ng/ml of EGF or TGFα. Cells were seeded in a T25 culture flask and housed in an incubator at 37°C, 100% humidity, 95% air/5% CO₂. Cells began to proliferate within 3-4 days and due to a lack of substrate lifted off the floor of the flask and continued to proliferate in suspension forming a hollow sphere of undifferentiated cells. After 6-7 days in vitro the proliferating clusters (neurospheres) were fed every 2-4 days by gentle centrifugation and resuspension in DMEM with the additives described above.

### EXAMPLE 6

After 6-7 days in vitro, individual cells in the neurospheres from Example 5 were separated by triturating the neurospheres with a fire polished pasteur pipette. Single cells from the dissociated neurospheres were suspended in tissue culture flasks in DMEM/F-12/10% hormone mix together with 20 ng/ml of EGF. A percentage of dissociated cells began to proliferate and formed new neurospheres largely composed of undifferentiated cells. The flasks were shaken well and neurospheres were allowed to settle in the bottom corner of the flask. The neurospheres were then transferred to 50 ml centrifuge tubes and centrifuged at 300 rpm for 5 minutes. The medium was aspirated off, and the neurospheres were resuspended in 1 ml of medium containing EGF. The cells were dissociated with a fire-narrowed pasteur pipette and triturated forty times. 20 microliters of cells were removed for counting and added to 20 microliters of Trypan Blue diluted 1:2. The cells were counted and re-plated at 50,000 cells/ml. Differentiation of cells was induced by maintaining the cells in the culture flasks in the presence of EGF or TGFα at 20 ng/ml without reinitiating proliferation by dissociation of the neurospheres or by plating on poly-ornithine in the continued presence of EGF or TGFα.

### EXAMPLE 7

Indirect immunocytochemistry was carried out with cells prepared as in Examples 1 and 2 which had been cultured for 14-30 days in vitro on glass coverslips. For anti-NSE (or anti-nestin) and anti-GFAP immunocytochemistry, cells were fixed with 4% paraformaldehyde in PBS and 95% ethanol/5% acetic acid, respectively. Following a 30 minute fixation period, coverslips were washed three times (10 minutes each) in PBS (pH = 7.3) and then incubated in the primary antiserum (NSE 1:300, nestin 1:1500 or GFAP 1:100) in PBS/10% normal goat serum/0.3% Triton-X-100) for two hours at 37°C. Coverslips were washed three times (10 minutes each) in PBS and incubated with secondary antibodies (goat-anti-rabbit-rhodamine for anti-NSE or anti-nestin and goat-anti-mouse-fluorescein for anti-GFAP, both at 1:50) for 30 minutes at 37°C. Coverslips were then washed three times (10 minutes each) in PBS, rinsed with water, placed on glass slides and coverslipped using Fluorsave, a mounting medium preferable for use with fluorescein-conjugated antibodies. Fluorescence was detected and photographed with a Nikon Optiphot photomicroscope.

### EXAMPLE 8

Rats were anesthetized with nembutal (25 mg/kg i.p) and injected with atropine sulfate (2 mg/kg i.p.). Animals sustained an ibotenate lesion of the striatum. 7 days after the lesion, the animals received an injection of cells prepared as in Examples 1 or 2 under stereotaxic control. Injections were made to the lesioned area via a 21-gauge cannula fitted with a teflon catheter to a microinjector. Injected cells were labelled with fluorescein-labelled microspheres. Animals were given behavioral tests before the lesion, after the lesion, and at various intervals after the. transplant to determine the functionality of the grafted cells at various post-operative time points, then killed and perfused transcardially with 4% buffered paraformaldehyde, 0.1% glutaraldehyde and 5% sucrose solution at 4°C. The brains were frozen in liquid nitrogen and stored at -20°C until use. Brains sections were sliced to 26 µm on a cryostat, fixed in 4% paraformaldehyde and stained using the M6 monoclonal antibody to stain for mouse neurons, and then reacted with a secondary anti-rat fluorescein-conjugated antibody. Neuronal and glial phenotype was identified by dual labelling of the cells with antibody to NSE and GFAP.

### EXAMPLE 9

Cells prepared according to Example 2 were cultured for 14-30 days in vitro on glass coverslips and then were fixed with 4% paraformaldehyde in PBS. Following a 30 minute fixation period, coverslips were washed three times (10 minutes each) in PBS (pH 7.3) and then incubated in the primary antiserum (anti-GABA 1:3000, anti-glutamine decarboxylase 1:500, and anti-substance P 1:100, in PBS containing 10% normal goat serum/0.3% Triton-X-100) for two hours at 37°C. Coverslips were washed three times (10 minutes each) in PBS and incubated with secondary antibodies (goat anti-rabbit or goat anti-sheep conjugated to fluorescein or rhodamine at 1:50) for 30 minutes at 37°C. Coverslips were then washed three times (10 minutes each) in PBS, rinsed with water, placed on glass slides and coverslipped using Fluorsave as the mounting medium. Fluorescence was detected and photographed with a Nikon Optiphot photomicroscope.

### EXAMPLE 10

Cells prepared as in Example 2 were centrifuged at 300 r.p.m. for 3 minutes, the medium aspirated and then the cells resuspended in warm DMEM/F-12 medium containing 10% glycerol at a concentration of approximately 1 x 10⁵ cells, ml, and slowly frozen to -20°C for 2 hours in a pre-cooled styrofoam box with the lid open. Once frozen, the cells were transferred to -80°C. The cells were thawed by placing the cryovial in a 37°C water bath, mixed by gentle agitation, an equal volume of warmed medium added to cells every 5 minutes for 15 minutes, and then the volume brought up to 10 ml. The cells were then centrifuged for 3 minutes at 300 rpm and the supernatant discarded. 10 microliters of cells were removed to determine viability and 10 microliters of cells were removed and stained with Trypan Blue and counted on a haemocytometer. Cells were resuspended in DMEM/F-12/10% hormone solution at 100,000 cells/flask and cultured 3-4 days at 37°C in T25 culture flasks.

### EXAMPLE 11

Cells are obtained from ventral mesencephalic tissue from a human fetus aged 8 weeks following routine suction abortion which is collected into a sterile collection apparatus. A 2 x 4 x 1 mm piece of tissue is dissected and dissociated as in Example 1. The cells are then cultured as in Example 5.

### EXAMPLE 12

Cells prepared as in Example 11 are used for neurotransplantation into a blood-group matched host with a neurodegenerative disease. Surgery is performed using a BRW computed tomographic (CT) stereotaxic guide. The patient is given local anesthesia supplemented with intravenously administered midazolam. The patient undergoes CT scanning to establish the coordinates of the region to receive the transplant. The injection cannula consists of a 17-gauge stainless steel outer cannula with a 19-gauge inner stylet. This is inserted into the brain to the correct coordinates, then removed and replaced with a 19-gauge infusion cannula that has been preloaded with 30 µl of tissue suspension. The cells are slowly infused at a rate of 3 µl/min as the cannula is withdrawn. Multiple stereotactic needle passes are made throughout the area of interest, approximately 4 mm apart. The patient is examined by CT scan post-operatively for hemorrhage or edema. Neurological evaluations are performed at various post-operative intervals, as well as PET scans to determine metabolic activity of the implanted cells.

### EXAMPLE 13

Neural tissue is obtained from the ventricular area by biopsy of a human subject and cultured as in Example 5.

### EXAMPLE 14

Cells proliferated as in Examples 1 or 2 are transfected with expression vectors containing the genes for the bFGF receptor or the NGF receptor. Vector DNA containing the genes are diluted in 0.1X TE (1 mM Tris pH 8.0, 0.1 mM EDTA) to a concentration of 40µg/ml. 220µl of the DNA is added to 250µl of 2X HBS (280 mM NaCl, 10 mM KCl, 1.5 mM Na₂HPO₄^{·}2H₂O, 12 mM dextrose, 50 mM HEPES) in a disposable, sterile 5 ml plastic tube. 31 µl of 2 M CaCl₂ is added slowly and the mixture is incubated for 30 minutes at room temperature. During this 30 minute incubation, the cells are centrifuged at 800g for 5 minutes at 4°C. The cells are resuspended in 20 volumes of ice-cold PBS and divided into aliquots of 1 x 10⁷ cells, which are again centrifuged. Each aliquot of cells is resuspended in 1 ml of the DNA-CaCl₂ suspension, and incubated for 20 minutes at room temperature. The cells are then diluted in growth medium and incubated for 6-24 hours at 37°C in 5%-7% CO². The cells are again centrifuged, washed in PBS and returned to 10 ml of growth medium for 48 hours.

### EXAMPLE 15

Cells transfected as in Example 14 are transplanted into a human patient as in Example 12.

## Claims

1. A method for the production *in vitro* of a cell culture of multipotent neural stem cells, said method comprising the steps of
(a) preparing or providing an initial culture medium comprising one or more growth factors capable of inducing multipotent neural stem cell proliferation, said one or more growth factors being selected from the group consisting of acidic fibroblast growth factor, basic fibroblast growth factor, epidermal growth factor, an EGF-like ligand, amphiregulin and transmitting growth factor alpha ;
(b) preparing a cell culture by adding to the culture medium of step (a) cells derived from mammalian neural tissue, said mammalian tissue not being obtained from an human embryo, and containing at least one multipotent neural stem cell capable of dividing without limit and producing progeny that are capable of differentiating into neurons and glia;
(c) proliferating said multipotent neural stem cell in said cell culture medium to produce neural stem cell progeny which include daughter multipotent neural stem cells and daughter cells which are capable of differentiating into neurons and glia ; and
(d) dissociating said neural stem cell progeny and transferring said neural stem cell progeny to fresh culture medium containing one or more growth factors capable of inducing multipotent neural stem cell proliferation , said one or more growth factors being selected from the group consisting of acidic fibroblast growth factor, basic fibroblast growth factor, epidermal growth factor, an EGF-like ligand, amphiregulin and transmitting growth factor alpha to proliferate said daughter multipotent neural stem cells and to produce more neural stem cell progeny.

2. The method according to claim 1, wherein the proliferation of said multipotent neural stem cell in step (c) or said daughter multipotent neural stem cells in step (d) occurs in a suspension culture.

3. The method according to claim 1, wherein the proliferation of said multipotent neural stem cell in step (c) or said daughter multipotent neural stem cells in step (d) occurs in a substrate-attached culture.

4. The method according to any one of claims 1 to 3 comprising perpetuation of multipotent neural stem cells by reinitiating proliferation of the daughter multipotent neural stem cells in step (d) by repeating step (d).

5. The method according to any one of claims 1 to 4, wherein said mammalian neural tissue comes from a human.

6. The method according to any one of claims 1 to 5, wherein said mammalian neural tissue comes from a fetus, neonate, juvenile or adult.

7. The method according to any one of claims 1 to 6, wherein the culture media used in steps (a) and (d) are selected from the group consisting of MEM, DMEM, RPMI, F-12 and a mixture of DMEM and F-12.

8. A method for producing *in vitro* differentiated neural cells derived from the neural stem cell progeny produced by the method of any one of claims 1 to 7 comprising inducing said daughter cells which are capable of differentiating to differentiate by plating said daughter cells on a fixed substrate or by allowing said daughter cells which are capable of differentiating to differentiate in suspension without reinitiating proliferation.

9. A method for drug screening of a putative therapeutic agent comprising applying said agent to the cell culture produced by the method of any one of claims 1 to 7 and monitoring the response of said cell culture.

10. A cell culture obtainable by the method of any one of claims 1 to 7 for use in treating neurological or neurodegenerative disease or CNS trauma, wherein said cell culture is derived from a source autologous to the intended recipient.

11. Use of a cell culture produced by the method of any one of claims 1 to 7 or the cell culture according to claim 10 in the manufacture of a medicament for treating neurological or neurodegenerative disease or CNS trauma.

12. Use of a cell culture according to claim 11 which is derived from a source autologous to the intended recipient.

13. Use of a cell culture according to claim 11 or 12, wherein the neurological or neurodegenerative disease is Parkinson's Disease, Alzheimer's Disease, Huntington's Chorea, schizophrenia, amytrophic lateral sclerosis, epilepsy, cerebral palsy or stroke or the neurodegenerative disease that causes ballismus or athetosis.

## Patentansprüche

1. Verfahren zur *in-vitro*-Herstellung einer Zellkultur multipotenter neuraler Stammzellen, wobei das Verfahren die folgenden Schritte umfasst:
(a) Zubereiten oder Bereitstellen eines anfänglichen Kulturmediums, umfassend einen oder mehrere Wachstumsfaktoren, die die Proliferation multipotenter neuraler Stammzellen induzieren können, wobei der/die eine oder mehreren Wachstumsfaktor(en) ausgewählt ist/sind aus der Gruppe bestehend aus saurem Fibroblastenwachstumsfaktor, basischem Fibroblastenwachstumsfaktor, epidermalem Wachstumsfaktor, einem EGF-ähnlichen Liganden, Amphiregulin und transformierendem Wachstumsfaktor alpha;
(b) Zubereiten einer Zellkultur durch Zugabe von Zellen zu dem Kulturmedium von Schritt (a), wobei die Zellen von neuralem Gewebe eines Säugers stammen, wobei das Säugergewebe nicht von einem menschlichen Embryo stammt, und wobei die Zellen mindestens eine multipotente neurale Stammzelle enthalten, die sich unbegrenzt teilen und Nachkommen produzieren kann, die in Neuronen und Glia differenzieren können;
(c) Proliferieren der multipotenten neuralen Stammzelle in dem Zellkulturmedium zur Herstellung neuraler Stammzellnachkommen, einschließlich multipotenter neuraler Tochterstammzellen und Tochterzellen, die in Neuronen und Glia differenzieren können; und
(d) Dissoziieren der neuralen Stammzellnachkommen und Transferieren der neuralen Stammzellnachkommen in ein frisches Kulturmedium, das einen oder mehrere Wachstumsfaktoren enthält, die die Proliferation multipotenter neuraler Stammzellen induzieren können, um die multipotenten neuralen Tochterstammzellen zu proliferieren und um weitere neurale Stammzellnachkommen zu produzieren, wobei der/die eine oder mehreren Wachstumsfaktor(en) ausgewählt ist/sind aus der Gruppe bestehend aus saurem Fibroblastenwachstumsfaktor, basischem Fibroblastenwachstumsfaktor, epidermalem Wachstumsfaktor, einem EGF-ähnlichen Liganden, Amphiregulin und transformierendem Wachstumsfaktor alpha.

2. Verfahren nach Anspruch 1, wobei die Proliferation der multipotenten neuralen Stammzelle in Schritt (c) oder der multipontenten neuralen Tochterstammzellen in Schritt (d) in einer Suspensionskultur stattfindet.

3. Verfahren nach Anspruch 1, wobei die Proliferation der multipotenten neuralen Stammzelle in Schritt (c) oder der multipotenten neuralen Tochterstammzellen in Schritt (d) in einer substratgebundenen Kultur stattfindet.

4. Verfahren nach einem der Ansprüche 1 bis 3, umfassend die Aufrechterhaltung der multipotenten neuralen Stammzellen durch Wiederbeginnen der Proliferation der multipotenten neuralen Tochterstammzellen in Schritt (d) durch Wiederholen von Schritt (d).

5. Verfahren nach einem der Ansprüche 1 bis 4, wobei das neurale Gewebe eines Säugers von einem Menschen stammt.

6. Verfahren nach einem der Ansprüche 1 bis 5, wobei das neurale Gewebe eines Säugers von einem Fötus, Neugeborenen, Jugendlichen oder Erwachsenen stammt.

7. Verfahren nach einem der Ansprüche 1 bis 6, wobei die in den Schritten (a) und (d) verwendete Kulturmedien ausgewählt sind aus der Gruppe bestehend aus MEM, DMEM, RPMI, F-12 und einem Gemisch aus DMEM und F-12.

8. Verfahren zur in-vitro-Herstellung von differenzierten neuralen Zellen, die aus den mit dem Verfahren nach einem der Ansprüche 1 bis 7 produzierten neuralen Stammzellnachkommen stammen, umfassend das Induzieren der Differenzierung der Tochterzellen, die sich differenzieren können, durch Ausplattieren der Tochterzellen auf ein gebundenes Substrat oder durch Ermöglichen der Differenzierung der Tochterzellen, die sich differenzieren können, in Suspension ohne Wiederbeginnen der Proliferation.

9. Verfahren zum Durchmustern eines Wirkstoffs eines mutmaßlichen therapeutischen Mittels, umfassend das Anwenden des Mittels auf die durch das Verfahren nach einem der Ansprüche 1 bis 7 produzierte Zellkultur und Überwachen der Antwort der Zellkultur.

10. Zellkultur, erhältlich durch das Verfahren nach einem der Ansprüche 1 bis 7 für die Verwendung zur Behandlung einer neurologischen oder neurodegenerativen Erkrankung oder eines ZNS-Traumas, wobei die Zellkultur von einer Quelle stammt, die autolog für den vorgesehenen Empfänger ist.

11. Verwendung einer durch das Verfahren nach einem der Ansprüche 1 bis 7 hergestellten Zellkultur oder der Zellkultur nach Anspruch 10 für die Herstellung eines Medikaments zur Behandlung einer neurologischen oder neurodegenerativen Erkrankung oder eines ZNS-Traumas.

12. Verwendung einer Zellkultur nach Anspruch 11, die von einer Quelle stammt, die autolog für den vorgesehenen Empfänger ist.

13. Verwendung einer Zellkultur nach Anspruch 11 oder 12, wobei die neurologische oder neurodegenerative Krankheit Parkinson-Krankheit, Alzheimer-Krankheit, Chorea Huntington, Schizophrenie, amyotropische Lateralsklerose, Epilepsie, Cerebralparese oder Schlaganfall oder die neurogenerative Erkrankung ist, die Ballismus oder Athetose verursacht.

## Revendications

1. Procédé pour la production in vitro d'une culture cellulaire de cellules souches neurales pluripotentes, ladite procédé comprenant les étapes consistant à :
a) préparer ou fournir un milieu de culture initial comprenant un ou plusieurs facteurs de croissance capable(s) d'induire la prolifération des cellules souches neuronales pluripotentes, lesdits un ou plusieurs facteurs de croissance est/sont choisi(s) dans le groupe comprenant un facteur de croissance acide des fibroblastes, un facteur de croissance basique des fibroblastes, un facteur de croissance épidermique, un ligand analogue à l'EGF, l'amphiréguline et le facteur de croissance transformant alpha,
b) préparer une culture cellulaire en ajoutant au milieu de culture de l'étape (a) des cellules dérivées de tissu neural de mammifère, ledit tissu de mammifère n'étant pas obtenu à partir d'un embryon humain, et contenant au moins une cellule souche neurale pluripotente capable de se diviser sans limite et de produire des descendants capables de se différencier en neurones et en glie ;
c) faire proliférer ladite cellule souche neurale pluripotente dans ledit milieu de culture cellulaire pour produire des descendants de cellule souche neurale qui comprennent des cellules souches neurales pluripotentes filles et des cellules filles qui sont capables de se différencier en neurones et en glie ; et
d) dissocier et transférer lesdits descendants de cellule souche neurale pour rafraîchir le milieu de culture contenant un ou plusieurs facteurs de croissance capable(s) d'induire la prolifération des cellules souches neurales, lesdits un ou plusieurs facteurs de croissance est/sont choisi(s) dans le groupe comprenant un facteur de croissance acide des fibroblastes, un facteur de croissance basique des fibroblastes, un facteur de croissance épidermique, un ligand analogue à l'EGF, l'amphiréguline et le facteur de croissance transformant alpha pour faire proliférer lesdites cellules souches neurales pluripotentes filles et pour produire plus de descendants de cellules souches neurales.

2. Procédé selon la revendication 1, dans lequel la prolifération de ladite cellule souche neurale pluripotente à l'étape (c) ou desdites cellules souches neurales pluripotentes à l'étape (d) se produit dans une culture en suspension.

3. Procédé selon la revendication 1, dans lequel la prolifération de ladite cellule souche neurale pluripotente à l'étape (c) ou lesdites cellules souches neurales pluripotentes filles à l'étape (d) se produit dans une culture sur substrat.

4. Procédé selon l'une quelconque des revendications 1 à 3, comprenant la perpétuation de cellules souches neurales pluripotentes en réinitiant la prolifération des cellules souches neurales pluripotentes filles à l'étape (d) en répétant l'étape (d).

5. Procédé selon l'une quelconque des revendications 1 à 4, dans lequel ledit tissu neural de mammifère provient d'un être humain.

6. Procédé selon l'une quelconque des revendications 1 à 5, dans lequel ledit tissu neural de mammifère provient d'un foetus, d'un nouveau-né, d'un enfant ou d'un adulte.

7. Procédé selon l'une quelconque des revendications 1 à 6, dans lequel les milieux de culture utilisés aux étapes (a) et (d) sont choisis dans le groupe comprenant le MEM, le DMEM, le RPMI, le F-12 et un mélange de DMEM et F-12.

8. Procédé pour produire in vitro des cellules neurales différenciées dérivées des descendants de cellules souches neurales produits par le procédé selon l'une quelconque des revendications 1 à 7, comprenant l'induction desdites cellules filles qui sont capables de se différencier pour effectuer une différenciation en plaquant lesdites cellules filles sur un substrat fixe ou en permettant auxdites cellules filles qui sont capables de se différencier, de se différencier en suspension sans réinitier la prolifération.

9. Procédé pour une évaluation préclinique médicament d'un agent thérapeutique putatif comprenant l'application dudit agent sur la culture cellulaire produite par le procédé selon l'une quelconque des revendications 1 à 7 et la surveillance de la réponse de ladite culture cellulaire.

10. Culture cellulaire pouvant être obtenue par le procédé selon l'une quelconque des revendications 1 à 7 destinée à être utilisée pour le traitement d'une maladie neurologique ou neurodégénérative ou d'un traumatisme du SNC, dans laquelle ladite culture cellulaire découle d'une source autologue au receveur prévu.

11. Utilisation d'une culture cellulaire produite par le procédé selon l'une quelconque des revendications 1 à 7 ou culture cellulaire selon la revendication 10, pour la fabrication d'un médicament pour traiter une maladie neurologique ou neurodégénérative ou un traumatisme du SNC.

12. Utilisation d'une culture cellulaire selon la revendication 11, qui est dérivée d'une source autologue au receveur prévu.

13. Utilisation d'une culture cellulaire selon la revendication 11 ou 12, dans laquelle la maladie neurologique ou neurodégénérative est la maladie de Parkinson, la maladie d'Alzheimer, la chorée de Huntington, la schizophrénie, la sclérose latérale amyotrophique, l'épilepsie, l'infirmité motrice cérébrale ou l'AVC ou la maladie neurodégénérative qui entraîne un ballisme ou une athétose.
